# EUROPEAN PATENT SPECIFICATION

(11) **EP 1 324 789 B1**
(45) Date of publication and mention of the grant of the patent: **03.05.2006**
(21) Application number: 01979630.9
(22) Date of filing: 10.10.2001
(51) Int. Cl.: A61M 5/00, A61M 5/145

(54) **Dental anesthesia syringe**
Spritze zur Dentalanästhesie
Seringue d'anesthésie dentaire

(30) Priority: 10.10.2000 US 238917 P; 12.10.2000 US 239714 P; 12.10.2000 US 239694 P
(43) Date of publication of application: 09.07.2003
(73) Proprietor: DENTSPLY INTERNATIONAL, INC., York, PA 17405-0872 (US)
(72) Inventor: HOHLFELDER, Ingrid, Elaine, Geneva, IL 60134 (US); ZDANOWSKI, Chester, L., Westmont, IL 60559 (US); PAPANEK, Tom, Lake Forest, IL 60015 (US); CHOU, Tan-Cheng, Arlington Heights, IL 60005 (US)
(74) Representative: Hartz, Nikolai
(86) International application number: PCT/US2001/031578
(87) International publication number: WO 2002/030490

(56) References cited:
- WO-A-01/87383
- US-A- 4 747 824
- US-A- 5 180 371
- US-A- 5 354 287

## Description

### TECHNICAL FILED

The present invention is directed toward a syringe for dispensing a fluid material for a dental anesthesia. The invention provides an electronic programmable device indicated for the injection of local anesthetics for infiltration and nerve block anesthesia administered prior to, or in conjunction with, dental procedures, and the like.

### BACKGROUND OF THE INVENTION

Syringes, of any type, are essentially positive displacement pumps which generate a flow of liquid.

With all syringe injections, the pressure in the tissue is a function of the flow rate into the tissue and the rate at which fluid can be absorbed into the tissue. A slow injection into soft tissue will generate only a few psi (pounds per square inch) of pressure. A rapid injection into the periodontal ligament space, which has limited capacity to absorb or distribute fluid, will result in high fluid pressure. In the latter case, the maximum fluid pressure will ultimately be limited by the pressure (force) attainable with the injection device. The present device provides a slow, controlled, injection which tends to reduce patient discomfort by reducing interstitial pressure (relative to traditional, manual, methods).

Dental anesthesia is known to be delivered from pre-filled, single use, carpules with a glass barrel and rubber-like plunger (by "rubber-like" it means actual rubber or some other material having physical properties similar to rubber). There are a variety of hand-operated dental syringes which hold these anesthesia carpules and drive the rubber plunger forward. In most clinical procedures, the dentist performs an aspiration to determine if a blood vessel has been entered, before injecting the anesthetic. (Injecting the anesthetic in the bloodstream is potentially hazardous.) Aspiration is accomplished by briefly retracting the carpule plunger to create a slight vacuum. There are a variety of means to retract the plunger, including the following: various mechanical hooks, harpoons, barbs, and corkscrews which embed in and grip the carpule plunger; a gripping member on the tip of the syringe plunger penetrates and grips the carpule's elastomeric seal. The gripping members are variously hooks, pointed needles, barbed needles, or corkscrews. This method is common in thumb-actuated syringes. One shortcoming of this method is that the carpule must be rapidly jammed onto the gripping member in order to embed it into the carpule seal without excess expulsion of anesthetic fluid. Another drawback is that various designs of gripping members either pull out of the carpule seal prematurely, or are not easily removed after injection is completed.

Sealed syringe plungers which create a vacuum behind the carpule plunger are known. A secondary seal on the syringe plunger creates a slight vacuum behind the carpule seal so the carpule seal retracts when the syringe plunger is pulled back. This method is used in a product known as The Wand computer controlled syringe. A drawback of this method is that the carpule seal is not consistently retracted. Another drawback is that the syringe plunger seal must be periodically cleaned, lubricated, or replaced.

Methods which create a vacuum in the carpule by distorting its needle septum are known. In this method, the syringe induces relative motion between the carpule and its seal, creating the slight vacuum. In a variation of this method, the syringe induces a deflection in the carpule's septum, creating a slight vacuum in the carpule.

It has been found in laboratory tests, that none of these methods work reliably, failing in one or more of the following ways: did not penetrate certain brands of carpules with high durometer rubber plungers; requiring excessive user effort or skill; pulling out of the carpule plunger and therefore failing to create an aspiration vacuum; and/or, becoming loaded with the silicone lubricant used on these carpule plungers, and then failing to grip the plunger.

A harpoon design which solves these problems is desirable.

Further, previous dental anesthesia syringes have had several problems in their operation: only a single injection rate for all procedures; only crude feedback on the amount of anesthetic injected; no feedback for the elapsed time of injection; does not allow the practitioner to speed-up the injection rate.

One device, described in U.S. Pat. No. 5,690,618, addressed some of these issues, but exhibited other problems: very complex to use, requiring the clinician to program various rates and times for each injection; and, no feedback for the elapsed time or cumulative volume of injection.

This present invention is indicated for all dental anesthesia procedures, including, but not limited to, procedures such as: periodontal ligament (PDL); intraseptal; posterior superior alveolar block (PSA); middle superior alveolar block (MSA); anterior superior alveolar block (infraorbital); maxillary block; greater palatine block (palaral); nasopalatine block; supraperiosteal (infiltration); inferior alveolar block (mandibular block); buccal block; mandibular block (Gow-Gates); intraseptal; intrapulpal; AMSA; P-AMSA, and the like.

The closest state of the art is represented by document US 5 180 371.

### DISCLOSURE OF THE INVENTION

It is therefore, an object of the present invention to provide syringe useful in dispensing dental anesthetic materials.

It is a further object of the invention to provide such a syringe that is computer controlled.

It is yet another object of the invention to provide such a syringe with an improved harpoon connector between a drive shaft and a carpule seal.

These and other objects of the invention that will become apparent from the following discussion are carried out by the invention as hereinafter described and claimed, whereby the invention is defined in claim 1.

In general, an electrically controlled syringe for dispensing a fluid material, comprises a power drive unit electrically and operatively connected to a syringe unit via at least one connecting conduit; said syringe unit having a releasably connected carpule holder, said carpule holder being initially loaded with the material to be dispensed, said carpule holder being releasably connected at one end to said syringe unit and fluidly connected at its other end to a dispensing needle, said carpule holder having a rubber-like plunger seal laterally displaceable therein between a dispensing and a retracted position, such that when said carpule plunger seal is moved toward a dispensing position, the material in said carpule holder is caused to flow through said dispensing needle; a longitudinally movable, powered drive shaft in said syringe unit and releasably connected to said carpule plunger seal, said drive shaft having an end proximal to and an end distal to said carpule plunger seal when said carpule holder is in place on said syringe unit; wherein said drive shaft is provided with a harpoon at said proximal end; said harpoon having a swept-back, barbed point and knife edges along its length, such that said barbed point is insertable into said rubber-like carpule plunger seal, and is selectively prevented from being removed by physical contact between said barbed point and said carpule plunger seal; an electric drive motor operatively affixed to said drive shaft, and operatively connected to said power drive unit, such that the rate of and direction of the longitudinal displacement of said drive shaft is selectable by electrical, operative signals received from said power drive unit to cause said drive motor to displace said drive shaft in a selected longitudinal direction at a selected rate, thereby laterally displacing said operatively connected harpoon and carpule plunger seal, said drive motor being operable with electric power received from said power drive unit; said harpoon being fabricated from a hard, corrosion resistant, sterilizable material; a stripper ring positioned within said syringe unit and proximate to said carpule plunger seal when said carpule holder is in place upon said syringe unit, such that when said carpule plunger seal is moved from the dispensing to the retracted position, said stripper ring physically engages said carpule plunger seal, preventing further retracting movement of said carpule plunger seal and hence, allowing extraction of said harpoon from said carpule plunger seal; said stripper ring having an inside diameter larger than said harpoon such that said harpoon is receivable therein; and at least one secondary injection control mechanism located proximate to said syringe unit and distal to said power drive unit, wherein said secondary injection control mechanism is operatively connected to said syringe drive motor to control the longitudinal displacement direction or rate of said drive shaft, and hence, the injection rate of the material dispensed from said needle.

An electrically controlled syringe for dispensing a fluid material comprises a power drive unit electrically and operatively connected to a syringe unit via at least one connecting conduit; said syringe unit having a releasably connected carpule holder, said carpule holder being initially loaded with the material to be dispensed, said carpule holder being releasably connected at one end to said syringe unit and fluidly connected at its other end to a dispensing needle, said carpule holder having a rubber-like plunger seal laterally displaceable therein between a dispensing and a retracted position, such that when said carpule plunger seal is moved toward a dispensing position, the material in said carpule holder is caused to flow through said dispensing needle; a longitudinally movable, powered drive shaft in said syringe unit and releasably connected to said carpule plunger seal, said drive shaft having an end proximal to and an end distal to said carpule plunger seal when said carpule holder is in place on said syringe unit; wherein said drive shaft is provided with a harpoon at said proximal end; said harpoon having a swept-back, barbed point and knife edges along its length, such that said barbed point is insertable into said rubber-like carpule plunger seal, and is selectively prevented from being removed by physical contact between said barbed point and said carpule plunger seal; an electric drive motor operatively affixed to said drive shaft, and operatively connected to said power drive unit, such that the rate of and direction of the longitudinal displacement of said drive shaft is selectable by electrical, operative signals received from said power drive unit to cause said drive motor to displace said drive shaft in a selected longitudinal direction at a selected rate, thereby laterally displacing said operatively connected harpoon and carpule plunger seal, said drive motor being operable with electric power received from said power drive unit; said harpoon being fabricated from a hard, corrosion resistant, sterilizable material; and at least one secondary injection control mechanism located proximate to said syringe unit and distal to said power drive unit, wherein said secondary injection control mechanism is operatively connected to said syringe drive motor to control the longitudinal displacement direction or rate of said drive shaft, and hence, the injection rate of the material dispensed from said needle.

A computer controlled syringe for dispensing a fluid material also comprises a power drive unit electrically and operatively connected to a syringe unit via at least one connecting conduit; said power drive unit including a logic control circuit; said syringe unit having a releasably connected carpule holder, said carpule holder being initially loaded with the material to be dispensed, said carpule holder being releasably connected at one end to said syringe unit and fluidly connected at its other end to a dispensing needle, said carpule holder having a rubber-like plunger seal laterally displaceable therein between a dispensing and a retracted position, such that when said carpule plunger seal is moved toward a dispensing position, the material in said carpule holder is caused to flow through said dispensing needle; a longitudinally movable, powered drive shaft in said syringe unit and releasably connected to said carpule plunger seal, said drive shaft having an end proximal to and an end distal to said carpule plunger seal when said carpule holder is in place on said syringe unit; wherein said drive shaft is provided with a harpoon at said proximal end; said harpoon having a swept-back, barbed point and knife edges along its length, such that said barbed point is insertable into said rubber-like carpule plunger seal, and is selectively prevented from being removed by physical contact between said barbed point and said carpule plunger seal; an electric drive motor operatively affixed to said drive shaft, and operatively connected to said power drive unit, such that the rate of and direction of the longitudinal displacement of said drive shaft is selectable by electrical, operative signals received from said logic control circuit of said power drive unit to cause said drive motor to displace said drive shaft in a selected longitudinal direction at a selected rate, thereby laterally displacing said operatively connected harpoon and carpule plunger seal, said drive motor being operable with electric power received from said power drive unit; said harpoon being fabricated from a hard, corrosion resistant, sterilizable material; and at least one secondary injection control mechanism located proximate to said syringe unit and distal to said power drive unit, wherein said secondary injection control mechanism is operatively connected to said syringe drive motor to control the longitudinal displacement direction or rate of said drive shaft, and hence, the injection rate of the material dispensed from said needle.

A programmable, electrically controlled syringe for dispensing a fluid material comprises a power drive unit electrically and operatively connected to a syringe unit via at least one connecting conduit; said syringe unit having a releasably connected carpule holder, said carpule holder being initially loaded with the material to be dispensed, said carpule holder being releasably connected at one end to said syringe unit and fluidly connected at its other end to a dispensing needle, said carpule holder having a rubber-like plunger seal laterally displaceable therein between a dispensing and a retracted position, such that when said carpule plunger seal is moved toward a dispensing position, the material in said carpule holder is caused to flow through said dispensing needle; a longitudinally movable, powered drive shaft in said syringe unit and releasably connected to said carpule plunger seal, said drive shaft having an end proximal to and an end distal to said carpule plunger seal when said carpule holder is in place on said syringe unit; wherein said drive shaft is provided with a harpoon at said proximal end; said harpoon having a swept-back, barbed point and knife edges along its length, such that said barbed point is insertable into said rubber-like carpule plunger seal, and is selectively prevented from being removed by physical contact between said barbed point and said carpule plunger seal; an electric drive motor operatively affixed to said drive shaft, and operatively connected to said power drive unit, such that the rate of and direction of the longitudinal displacement of said drive shaft is selectable by electrical, operative signals received from said power drive unit to cause said drive motor to displace said drive shaft in a pre-selected longitudinal direction at a pre-selected rate, thereby laterally displacing said operatively connected harpoon and carpule plunger seal, said drive motor being operable with electric power received from said power drive unit; said harpoon being fabricated from a hard, corrosion resistant, sterilizable material; and at least one secondary injection control mechanism located proximate to said syringe unit and distal to said power drive unit, wherein said secondary injection control mechanism is operatively connected to said syringe drive motor to control the longitudinal displacement direction or rate of said drive shaft, and hence, the injection rate of the material dispensed from said needle.

An electrically controlled syringe for dispensing a fluid material comprises a power drive unit electrically and operatively connected to a syringe unit via at least one connecting conduit; said syringe unit having a releasably connected carpule holder, said carpule holder being initially loaded with the material to be dispensed, said carpule holder being releasably connected at one end to said syringe unit and fluidly connected at its other end to a dispensing needle, said carpule holder having a rubber-like plunger seal laterally displaceable therein between a dispensing and a retracted position, such that when said carpule plunger seal is moved toward a dispensing position, the material in said carpule is caused to flow through said dispensing needle; a longitudinally movable, powered drive shaft in said syringe unit and releasably connected to said carpule plunger seal, said drive shaft having an end proximal to and an end distal to said carpule plunger seal when said carpule holder is in place on said syringe unit; wherein said drive shaft is provided with a harpoon at said proximal end, said harpoon having a swept-back, barbed point and knife edges along its length, such that said barbed point is insertable into said rubber-like carpule plunger seal, and is selectively prevented from being removed by physical contact between said barbed point and said carpule plunger seal; an electric drive motor operatively affixed to said drive shaft, and operatively connected to said power drive unit, such that the rate of and direction of the longitudinal displacement of said drive shaft is selectable by electrical, operative signals received from said power drive unit to cause said drive motor to displace said drive shaft in a selected longitudinal direction at a selected rate, thereby laterally displacing said operatively connected harpoon and carpule plunger seal, said drive motor being operable with electric power received from said power drive unit; said harpoon being fabricated from a hard, corrosion resistant, sterilizable material; said power drive unit having image displays to provide digital or analog indicia of system parameters selected from the group consisting of elapsed time of dispensing, rate of dispensing, volume of material dispensed, dispensing or aspirating mode, or combinations thereof.

In another alternative a syringe comprises an electrically controlled syringe for dispensing a fluid material having a power drive unit electrically and operatively connected to a syringe unit via at least one connecting conduit; said syringe unit having a releasably connected carpule holder, said carpule holder being initially loaded with the material to be dispensed, said carpule holder being releasably connected at one end to said syringe unit and threadably and fluidly connected at its other end to a dispensing needle, said carpule holder having a rubber-like plunger seal laterally displaceable therein between a dispensing and a retracted position, such that when said carpule plunger seal is moved toward a dispensing position, the material in said carpule holder is caused to flow through said dispensing needle; a longitudinally movable, powered drive shaft in said syringe unit and releasably connected to said carpule plunger seal, said drive shaft having an end proximal to and an end distal to said carpule plunger seal when said carpule holder is in place on said syringe unit; wherein said drive shaft is provided with a harpoon at said proximal end; said harpoon having a swept-back, barbed point and knife edges along its length, such that said barbed point is insertable into said rubber-like carpule plunger seal, and is selectively prevented from being removed by physical contact between said barbed point and said carpule plunger seal; an electric drive motor operatively affixed to said drive shaft, and operatively connected to said power drive unit, such that the rate of and direction of the longitudinal displacement of said drive shaft is selectable by electrical, operative signals received from said power drive unit to cause said drive motor to displace said drive shaft in a selected longitudinal direction at a selected rate, thereby laterally displacing said operatively connected harpoon and carpule plunger seal, said drive motor being operable with electric power received from said power drive unit; said harpoon being fabricated from a hard, corrosion resistant, sterilizable material; a stripper ring positioned within said syringe unit and proximate to said carpule plunger seal when said carpule holder is in place upon said syringe unit, such that when said carpule plunger seal is moved from the dispensing to the retracted position, said stripper ring physically engages said carpule plunger seal, preventing further retracting movement of said carpule plunger seal and hence, allowing extraction of said harpoon from said carpule plunger seal; said stripper ring having an inside diameter larger than said harpoon such that said harpoon is receivable therein; and at least one secondary injection control mechanism located proximate to said syringe unit and distal to said power drive unit, wherein said secondary injection control mechanism is operatively connected to said syringe drive motor to control the longitudinal displacement direction or rate of said drive shaft, and hence, the injection rate of the material dispensed from said needle.

A system for administering an anesthetic into a patient, comprises a container storing an anesthetic; a needle; and means for delivering the anesthetic through the needle at a first predetermined rate for a first predetermined time and then at a second predetermined rate for a second predetermined time.

Another system for delivering an anesthetic into a patient comprises a handheld unit comprising a needle, a cartridge of anesthetic and means for flowing the anesthetic through the needle; and, a control unit to control operation of the handheld unit, the control unit comprising means for operating the means for flowing the anesthetic through the needle to deliver the anesthetic through the needle at a first predetermined rate for a first predetermined time and then at a second predetermined rate for a second predetermined time, wherein the second predetermined rate is selectable by a user. The means for flowing the anesthetic may comprise a motor and a plunger, wherein operation of the motor in a first direction advances the plunger against the cartridge of anesthetic to flow anesthetic from the cartridge through the needle and operation of the motor in a second direction retracts the plunger from the cartridge of anesthetic; and, the means for operating the means for flowing further comprises a processing unit to generate control signals to operate the motor to advance and retract the plunger.

A system comprises a computer program embodied on a computer readable medium and executable by a microprocessor for controlling a device to deliver a local anesthetic into a patient through a needle, the computer program product comprises computer instructions for executing the steps of: generating a first at least one signal to operate the device to deliver the local anesthetic at a first predetermined rate for a first predetermined time period; transmitting the first at least one signal to the device to operate the device to deliver the local anesthetic at a first predetermined rate for a first predetermined time period; generating a second at least one signal to operate the device to deliver the local anesthetic at a second predetermined rate for a second predetermined time period; and, transmitting the second at least one signal to the device for the device to operate at the second predetermined rate for the second predetermined time immediately upon completion of the first predetermined time period. The second predetermined rate may be in the range of 0.005 - 0.02 cc/sec. The second predetermined time may be in the range of 30 - 240 seconds. The step of generating a second at least one signal may further comprise the steps of receiving a user signal corresponding to an injection type selected by a user; determining a delivery rate and delivery time based on the injection type received in the user signal; and, generating the second at least one signal to operate the device to deliver the local anesthetic at the delivery rate for the delivery time based on the selected injection type. The computer program may include instructions for executing the steps of calculating a rate of delivery of the local anesthetic through the needle, and displaying the calculated rate of delivery of the local anesthetic. The program may also include instructions for calculating an elapsed time of delivery of the local anesthetic through the needle and displaying the elapsed time of delivery of the local anesthetic; or, calculating a volume of the local anesthetic delivered through the needle and displaying the volume of delivered local anesthetic. The computer program may also include instructions for receiving a user signal corresponding to a load command selected by a user; generating a third at least one signal to operate the device to position components of the device to receive a cartridge storing the local anesthetic; and, transmitting the third at least one signal to the device for loading the local anesthetic cartridge in the device. It may also include instructions for receiving a user signal corresponding to an unload command selected by a user; generating a third at least one signal to operate the device to position components of the device to remove a cartridge storing the local anesthetic; and, transmitting the third at least one signal to the device for unloading the local anesthetic cartridge from the device. The program may include instructions for receiving a user signal corresponding to a double command selected by a user, generating a third at least one signal to operate the device to increase the rate of delivery of the local anesthetic to twice the rate of delivery being provided by the device; and, transmitting the third at least one signal to the device for increasing the rate of delivery of the local anesthetic by the device.

There is also described a method of delivering a local anesthetic to a patient. The method comprises the steps of selecting a preprogrammed injection type for delivering a local anesthetic to a patient; delivering the local anesthetic to a patient with a motor driven syringe at a first predetermined rate for a first predetermined time period based on the selected injection type; then delivering the local anesthetic to a patient with the motor driven syringe at a second predetermined rate for a second predetermined time period based on the selected injection type. In one embodiment, the step of delivering the local anesthetic to a patient with a motor driven syringe at a first predetermined rate further comprises generating in a control unit a first at least one signal to operate the motor driven syringe to deliver the local anesthetic to a patient at a first predetermined rate for a first predetermined time period; and, transmitting from the control unit to the motor driven syringe the first at least one signal to operate the motor driven syringe to deliver the local anesthetic at a first predetermined rate for a first predetermined time period. The step of delivering the local anesthetic to a patient with the motor driven syringe at a second predetermined rate may further comprise generating in the control unit a second at least one signal to operate the motor driven syringe to deliver the local anesthetic at a second predetermined rate for a second predetermined time period; and, transmitting from the control unit to the motor driven syringe the second at least one signal for the motor driven syringe to operate at the second predetermined rate for the second predetermined time immediately upon completion of the first predetermined time period.

### BRIEF DISCUSSION OF THE DRAWINGS

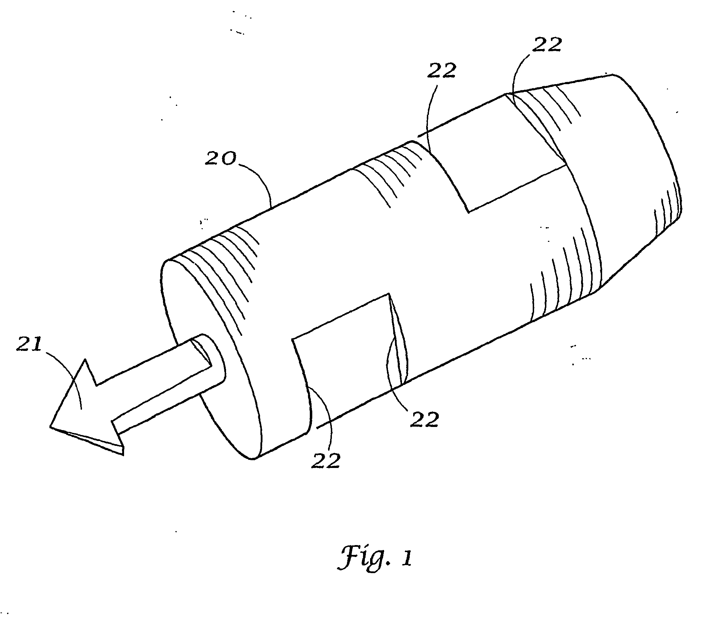

Fig. 1 is a perspective view of the harpoon portion of a dental syringe, according to the invention.

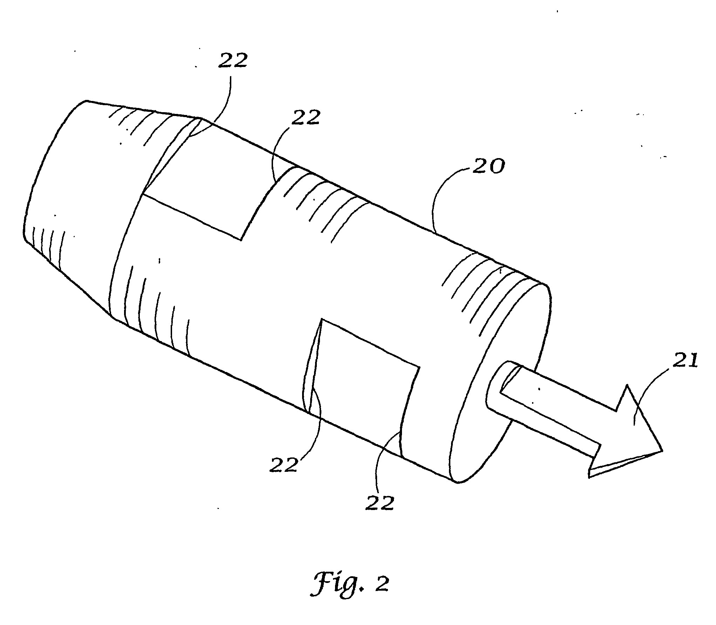

Fig. 2 is a view of the opposite side of the harpoon as shown in fig. 1.

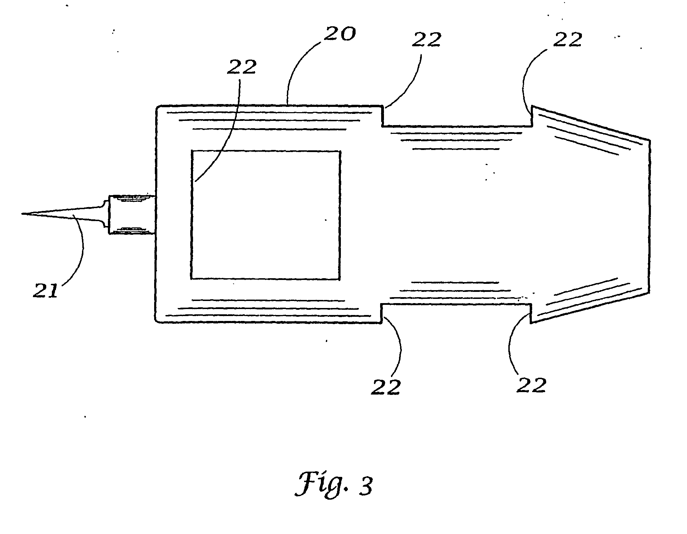

Fig. 3 is a side elevational view of the harpoon shown in Fig. 1.

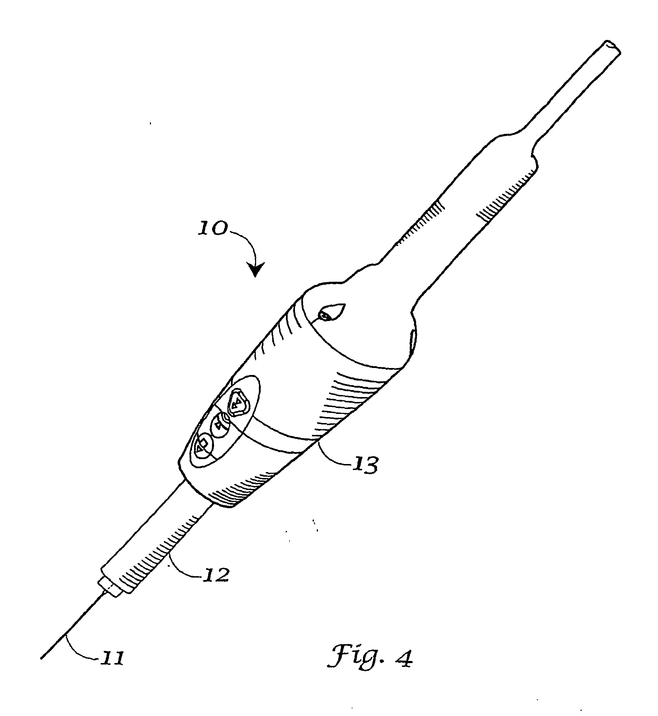

Fig. 4 is a perspective view of a dental syringe according to the present invention.
Fig. 5 is a lengthwise cross-sectional view of the syringe of Fig. 4.

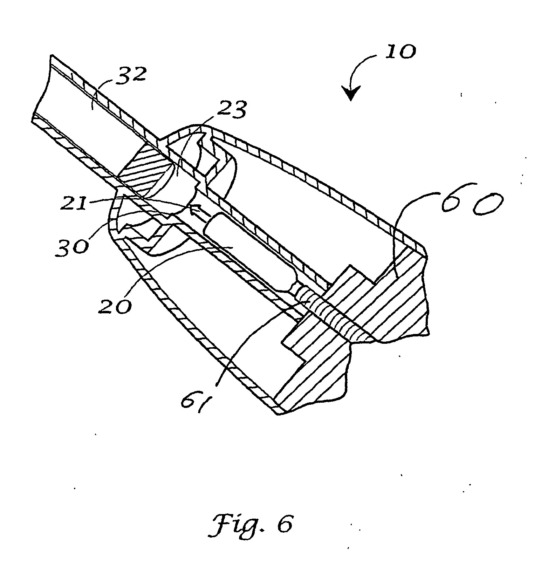

Fig. 6 is a cross-sectional, perspective view of one portion of the syringe of Fig. 5.

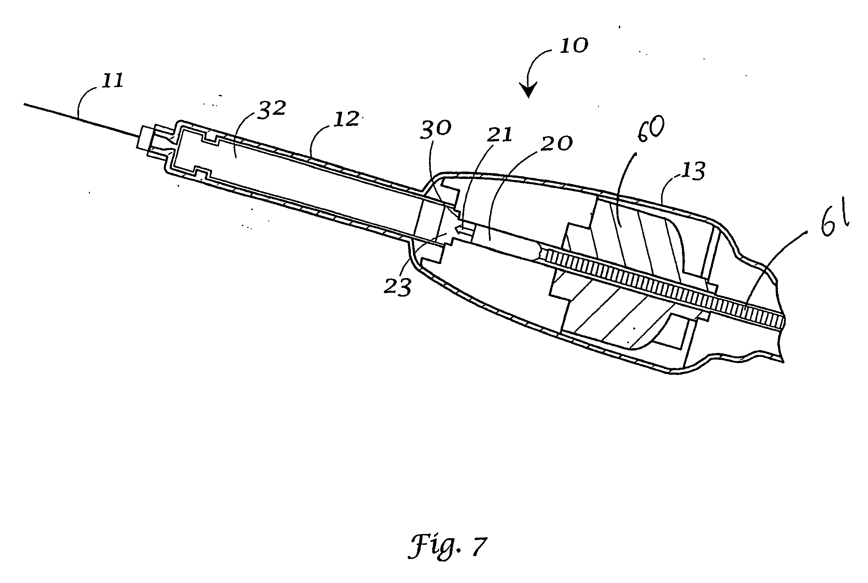

Fig. 7 is a closeup view of one portion of the cross-section of Fig. 5, showing the harpoon of Figs. 1-3 in place in the syringe.
Fig. 8 is a partially schematic representation of a control panel for the computer-controlled syringe according to the present invention.
Fig. 9 is a perspective view of a carpule holder useful with the syringe of Fig. 4.
Fig. 10 is a side elevational view of the carpule holder of Fig. 9.
Fig. 11 is a sectional view of the carpule holder of Fig. 10 taken along lines 11-11 thereof.
Fig. 12 is a closeup sectional view of one portion of the carpule holder of Fig. 10, taken along lines 12-12 thereof
Fig. 13 is an end elevational view of the carpule holder of Fig. 10.
Fig. 14 is a sectional, partially broken away view of carpule of Fig. 14, taken along lines 14-14 thereof
Fig. 15 is a perspective view of the syringe of Fig. 4 without the carpule holder in place and showing the carpule holder-connector end.

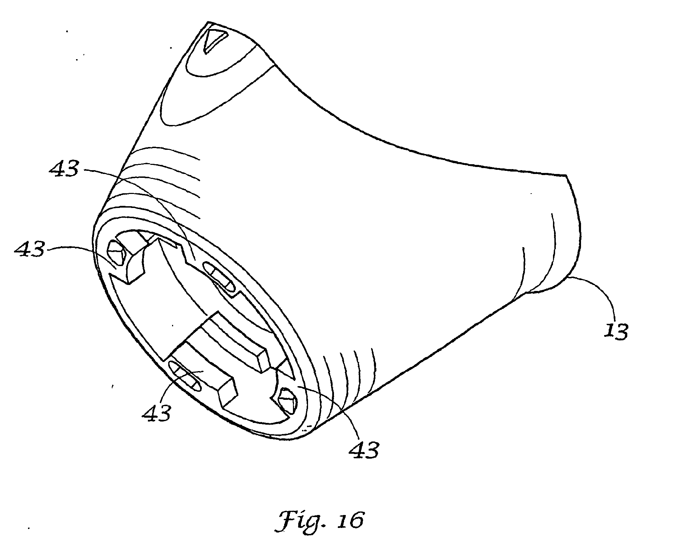

Fig. 16 is a closeup view of one end of the syringe of Fig. 15.
Fig. 17 is a flowchart of one embodiment of the programming logic employed with the computer controlled device of Fig. 1.

### PREFERRED EMBODIMENTS FOR CARRYING OUT THE INVENTION

An exemplary computer controlled syringe, embodying the concepts of the present invention, is generally shown by the number 10 on the attached drawings. Syringe 10 has a dispensing tip 11 fluidly affixed to a carpule holder 12, which carpule holder 12 is releasably affixed or connected to a syringe power unit 13.

Carpule holder 12 is initially (that is, prior to dispensing) loaded with the material to be dispensed (not shown) by any conventional means, such as a conventional carpule or the like. Any carpule capable of being dispensed by the action of a physically engaging plunger (to be discussed below) is within the scope of the invention. Carpule holder 12 may be affixed to syringe 10 by any conventional means, including for example, bayonet connector 50 at one end of carpule holder 12. At it other end, carpule holder 12 is preferably provided with means to affix or removably affix the dispensing tip 11. In the case of the use of syringe 10 to dispense a dental anesthetic or the like, dispensing tip 11 is a hypodermic needle, which is affixed by conventional means, such as friction, screw threads or the like, to carpule holder 12. Preferably, dispensing tip 11 is fluidly affixed to carpule holder 12, so as to fluidly communicate with the interior thereof, or whatever carpule or the like is employed.

Carpule holder 12 is provided with a carpule plunger seal 32, which is preferably rubber-like in manufacture, for reasons to be discussed. Carpule plunger seal 32 is preferably laterally displaceable within carpule holder 12 to thereby provide for dispensing of material from carpule holder 12 or aspiration of external material through dispensing tip 11. Thus, preferably, carpule plunger seal 32 is selectively, laterally displaceable between a dispensing and a retracting movement. When carpule plunger seal 32 is caused to move toward affixed dispensing tip 11, material in carpule holder 12 is caused to flow toward dispensing tip 11, and when expressed therethrough, is said to have dispensed the material.

Syringe power unit 13 of syringe 10 is preferably provided with an electric drive motor 60, which is employed to laterally displace a drive shaft 61. Motor 60 may be of any conventional design, but is preferably an electrically powered stepper motor with integral internal rotating nut that drives a lead-screw to provide open-loop linear motion. Such motors are commercially available for example, from Haydon Switch and Signal, as well as others. Further, motor 60 should be capable of being controlled as to start and stop of motion, as well as amount of and speed of the lateral displacement of the drive shaft 61, by signals received from an operator, and more preferably received via conduit 41 from power drive unit 40. Motor 60 may also be powered by electricity received through conduit 41 or by any other conventional means, such as batteries located in syringe 10 (not shown). Drive shaft 61 is operatively and releasably connected to carpule plunger seal 32, by any means but preferably by the means described herein.

Drive shaft 61 preferably has an end proximal to and an end distal to carpule holder 12 when carpule holder 12 is connected to syringe 10. In order to effect the connection between drive shaft 61 and carpule plunger seal 32, it is preferred to employ an harpoon 20 that will be hereinafter described.

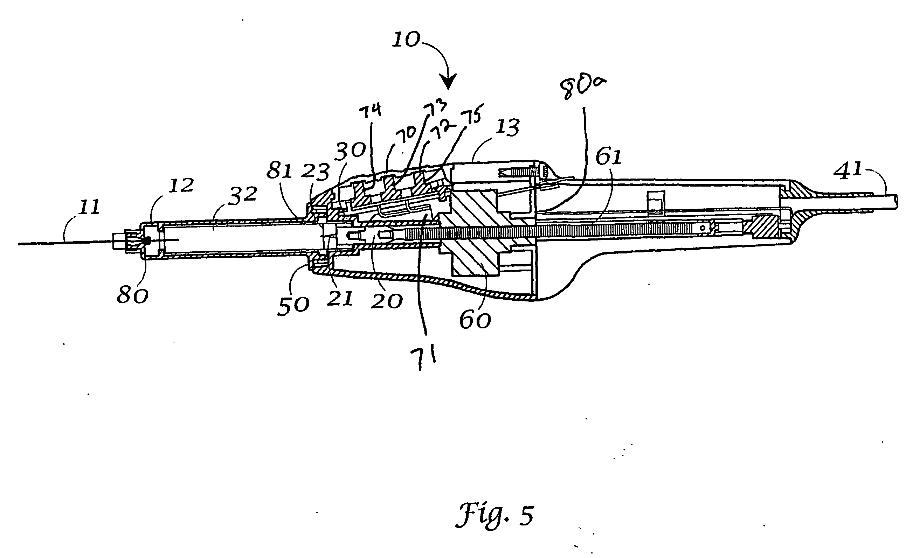

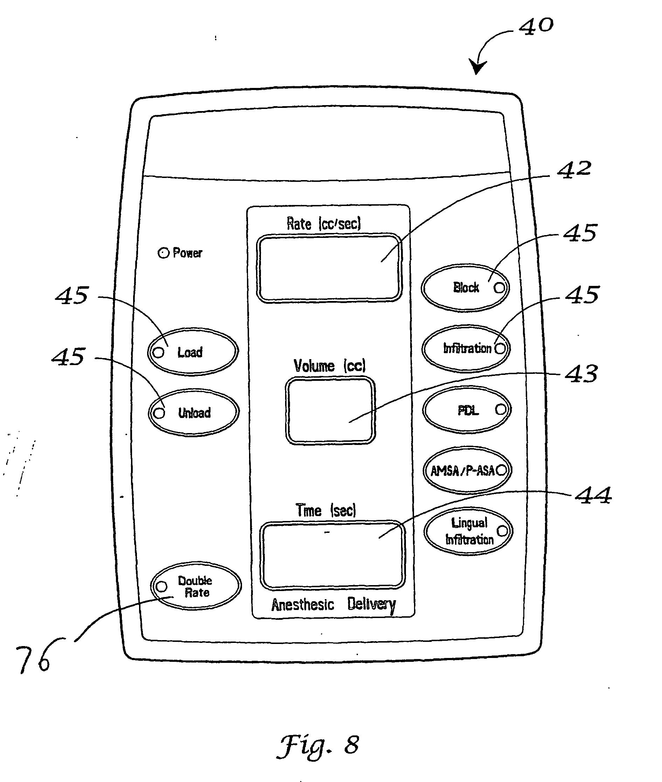

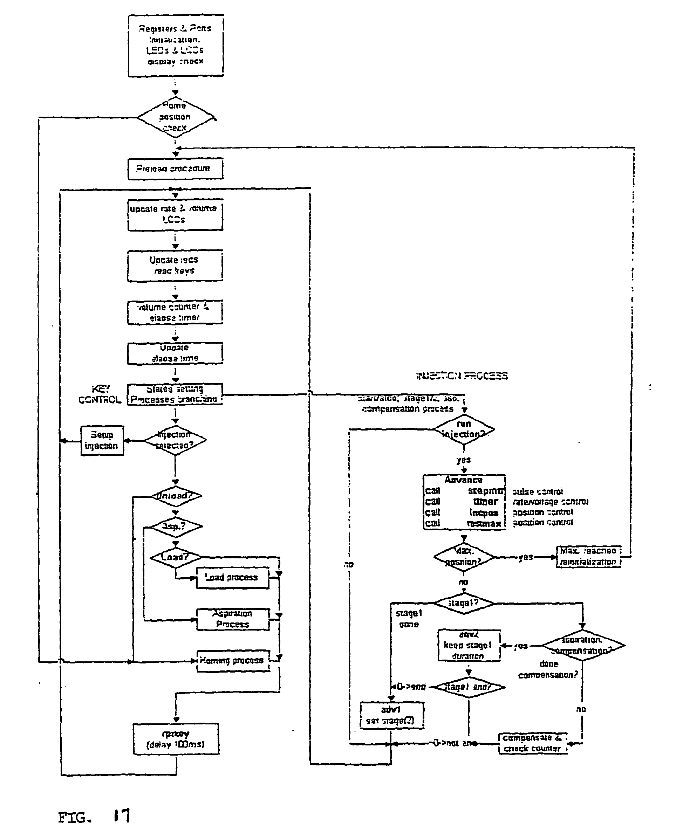

Syringe 10 is operatively and electrically connected to a power drive or base unit 40 (Fig. 8) via a connecting conduit 41 (Figs. 5). Power drive unit 40 via conduit 41 provides electrical signal to syringe 10 to control the operation thereof, and preferably includes a logic control circuit (not shown) of any suitable sort to provide such selected or predetermined control signals, as exemplified by the flowchart shown in Fig. 17. For example, power drive unit 40 may be used to control the flow rate, flow duration, start, stop, elapsed time, volume of dispensed material, direction of material flow, connection to a carpule (known as loading) or disconnecting therefrom (unloading) or the like. The mechanism of such controls will be described below in greater detail. Control signals from power drive unit 40 may be digital or analog, and may be displayed by any suitable means, including using digital readouts 42 (rate of dispensing), 43, (volume of material dispensed), 44 (time of dispensing), or any other desired parameter without limitation. Control mechanisms include buttons 45 for controlling dispensing conditions or parameters, or the like. Power drive unit 40 may be preset for automatic control of dispensing parameters, or such parameters may be individually controlled. As an example of a preset parameter, a button 45 may provide for a doubling of the rate of dispensing of material. By using a logic control circuit or computer, the number of, type of, rate of or the li9ke of all syringe parameters can be preselected, and hence, the device is programmable.

Conduit 41 may also be used to provide electrical power to syringe 10 for purposes to be more fully explained in the following discussion. If required, multiple conduits (not shown) similar to conduit 41 or of some other conventional design, may be employed.

Syringe 10 will be exemplified herein with respect to the dispensing of a dental anesthetic material.

The general operation of syringe 10 in delivering anesthetics to a patient, is well known in the art, except as otherwise described, noted and claimed. For example, a power driven syringe is shown in U.S. Pat. No. 5,690,618.

As stated above, a harpoon 20 is provided according to the present invention. Harpoon 20 would typically and preferably be made of stainless steel or other hard, corrosion resistant, sterilizable, material. Harpoon 20 has a unique barb 21 geometry and hardened, knife-like, edges 22 located along its length, with the following advantages: the thin configuration, sharp point, and hard knife edges 22 of the harpoon uniquely allow consistent penetration into all types of elastomer carpule seals, such as carpule plunger seal end 23, even those of hard rubber, with lower force than other gripper types. Thus, it does not require the operator to jam the carpule into the syringe. The swept-back barbs 21 allow the harpoon to consistently remain in the carpule plunger, up to five times more effectively than other gripper types. Thus, aspiration is consistent. This design is easily fabricated at low cost, and has a long life (number of insertions into rubber). The design reduces or eliminates the drawbacks of the vacuum type.

Because harpoon 20 is affixed to carpule plunger seal 32 and to drive shaft 61, lateral displacement of drive shaft 61 also causes lateral displacement of harpoon 20 and hence, also of affixed carpule plunger seal 32.

Another feature of the invention is the incorporation of a stripper ring 30 in the syringe device 10. This ring has an inside diameter (ID) slightly larger than the harpoon 20 but smaller than a standard carpule plunger seal 23. Thus, when the syringe plunger seal 32 is fully retracted, the harpoon 20 is pulled out of the carpule seal 23 while the carpule seal 23 remains within the carpule holder 12, by physical contact between stripper ring 30 and carpule plunger seal 32, thereby preventing further movement of carpule plunger seal 32. Continued retracting movement of drive shaft 61 and affixed harpoon 20 past the point physical contact between stripper ring 30 and carpule plunger seal 32 will cause harpoon 20 to be physically disengaged from carpule plunger seal 32. By being a ring of proper dimension, harpoon 20 and drive shaft 61 maybe received within stripper ring 30.

Harpoon 20 has the following advantages: it readily penetrates the carpule plunger seal 32 with only moderate force, even plungers made of high durometer rubber; it does not pull out of the carpule plunger seal 32, even in repeated aspirations; nonetheless, it can be readily stripped from the carpule plunger seal 32 with proper syringe design, using stripper ring 30; its shape is suited to low-cost manufacturing.

As discussed above, it is preferred to control dispensing parameters of syringe 10 by control signals or commands received from power drive unit 40. It is also an advantage of the present invention, that an operator can control some or all such parameters by use of a secondary injection control mechanism 70 (Fig. 5) located proximate to syringe 10 and distal to power drive unit 41. Secondary injection control mechanism 70 is operatively connected to said drive motor 60, such as by connector 71, to control the longitudinal displacement direction or rate of drive shaft 61, by command signals that start, stop, adjust speed, torque, or the like of motor 60, and hence, the injection rate of the material dispensed from dispensing tip 11. Secondary injection control mechanism 70 may be of any design such as a switchboard 72, button 73 design as depicted in the drawings.

According to one embodiment of the invention, a limit switch of any suitable design, including those of analog or digital design is employed. For example, limit switch 80a (Fig. 5) may be operatively connected to drive motor 60. A limit (or "home") switch 80a is actuated when the leadscrew or drive shaft 61 is in the fully retracted position. Preferably, the unit 10 automatically retracts the drive shaft 61 to home position upon power up. All drive shaft 61 motion is then referenced by counting motor steps from this position. Previous syringe devices did not have a limit switch, but relied on running the motor in reverse for a fixed time at power up to ensure it would reach home position.

Certain injection modes, such as PDL injections, require increased injection pressure. When the PDL injection type is selected, motor voltage is preferably increased to 10 volts during the injection Slow Phase and Regular Phase forward motion. Other injection types and other plunger motions are at normal motor voltage of 5 volts. To prevent motor overheating, voltage is increased for only 10% duty cycle.

The syringe 10 according to the present invention also divides the delivery of anesthesia into two phases. According to a described method, during the first 10 seconds of the injection, anesthetic is delivered at an extremely slow rate to maximize patient comfort. The injection rate then automatically increases to the preprogrammed rate associated with the injection type you have selected. The following steps refer to the control panel of Fig. 8.
A. Select your injection by depressing the appropriate button under *Injection Technique.* The injection rate will be displayed in the box title *Rate cc*/*sec.*
B. Once the injection technique is selected, you are ready to proceed.
C. Aspiration is achieved by pressing and releasing the middle button on the handpiece, once.
D. After aspiration, press an release the front *start*/*stop button* on the handpiece to initiate the injection.
E. At any time during the injection you may stop by simply pressing the front *start*/*stop button* on the handpiece to stop the program.
F. You can double the rate of injection at any time by pressing the *back button* on the handpiece or the *double rate button* on the base unit. To turn this feature off, simply push the Double button again on either the handpiece or the control box.
G, When you are through with the injection, press and release the front *start*/*stop button* once to stop the program.
H. If you inject into a new site, change your injection technique setting if necessary, follow steps A-D and the program will automatically start over.
I. When finished, re-sheath the needle and set the handpiece in the holder."

Other physical embodiments utilizing the same simplified control scheme. For example, a unitary battery operated handpiece.

It will be appreciated that the syringe according to the present invention is simple to use. Control choices are directly related to known clinical practice rather than arcane rates and times. It provides clinically useful display information.

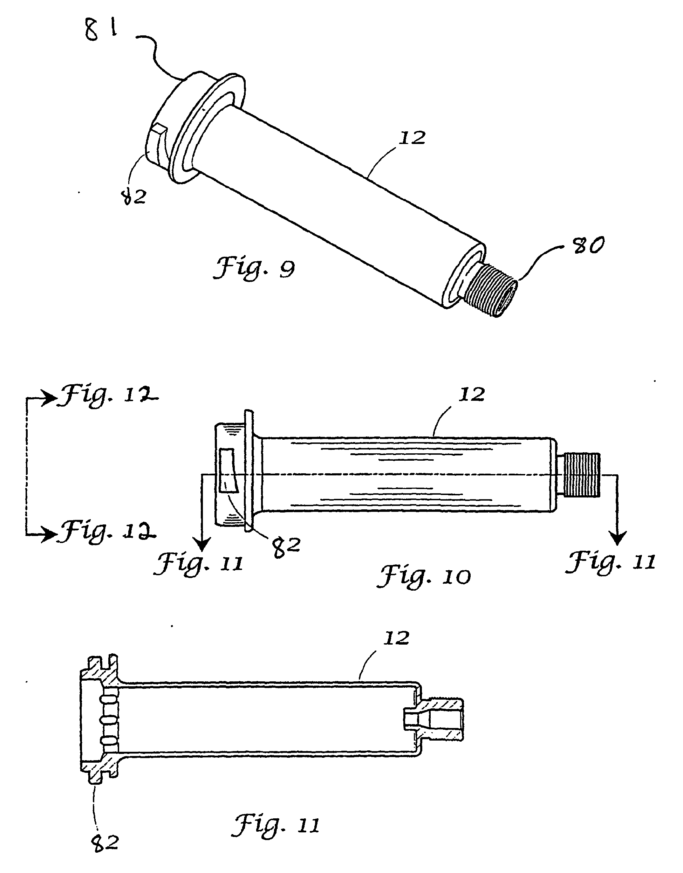

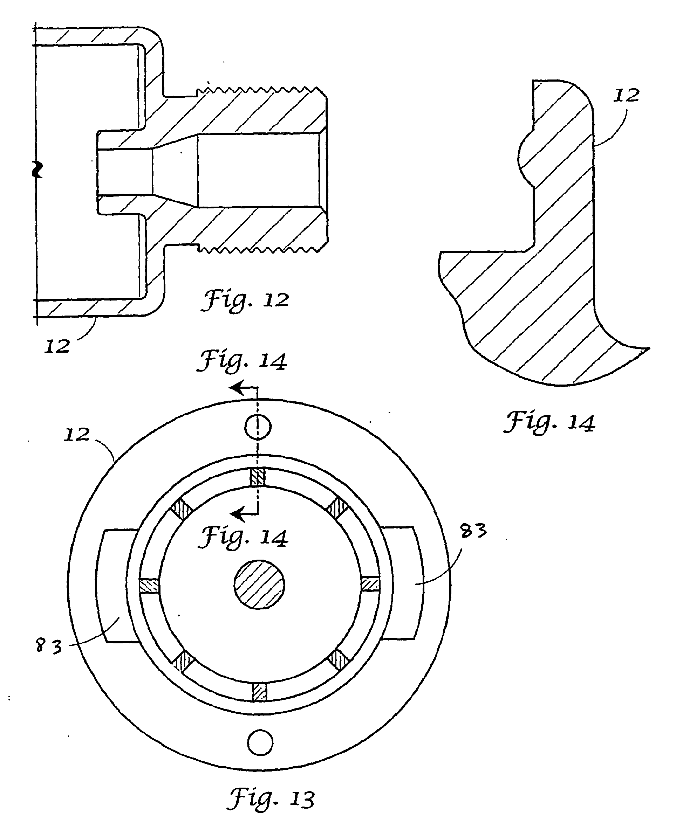

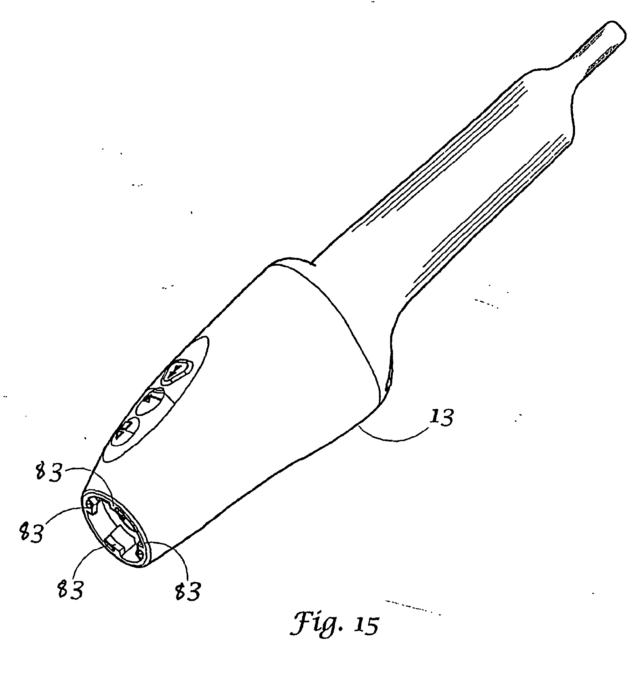

As shown in Fig. 5 and 9, carpule holder 12 is provided with a discharge end 80 and a connector end 81. Connector end 81 is used to removably affix carpule holder 12 to syringe 10. Connector end 81 is provided with wedge lugs 82 (Figs. 9-11), which physically engage circumferential lips 83 carried by syringe 10 (Figs. 13 and 15). The physical engagement of lugs 82 with lips 83 causes lips 83 to removably hold carpule holder12 thereto, in a bayonet-like manner.

An exemplary computer controlled syringe, embodying the concepts of the present invention, is generally shown by the number 10 on the attached drawings. Syringe 10 has a needle 11 affixed to a carpule 12, which carpule 12 is affixed to a power drive unit 13 (Fig. 5). The general operation of syringe 10 in delivering anesthetics to a patient, is well known in the art, except as otherwise described, noted and claimed. For example, a power driven syringe is shown in U.S. Pat. No. 5,690,618.

As described herein, the present inventive syringe is a partially automated, mechanized system intended for injection of anesthetics in dental procedures. The intended benefit of this automation is precise control of anesthetic injections, resulting in less undesired numbing of the patient's facial muscles, reduced patient pain, easier injection into hard tissue areas, and improved practitioner comfort.

The system is comprised of an electronic control unit, a handheld motor driven syringe (the "handpiece"), and a single-use cartridge holder. All operating displays and the majority of the operator controls are located on the front panel of the control unit. The control unit also houses a microprocessor and motor drive circuits. The handpiece houses a stepper motor and lead screw which drive the syringe plunger. The single-use cartridge holder encloses standard 1.8 cc dental anesthetic cartridges and mates with standard dental anesthetic needles.

In preparation for use, the dental practitioner assembles a needle to the cartridge holder, places a standard 1.8cc cartridge of anesthetic in a disposable plastic cartridge holder, and attaches the cartridge holder to the handpiece. The practitioner then sets operating parameters on the control unit. After the practitioner inserts a standard dental needle into the patient, injection of anesthetic is regulated by a combination of controller programming and practitioner actuation of buttons on the handpiece or controller.

The inventive syringe according to the invention uses a two-stage injection to provide you an easier means of delivering an effective pain controlled injection with as minimal post-op discomfort as possible. The following describes method steps of using the present syringe. Where appropriate, reference is made to the drawings, it being understood that the structure described is that as was above discussed. Structure not shown in the drawings may be alluded to, in which case it is understood that the same is not shown.

### POWERING UP

1. Plug the AC power cord (not shown) of the external power supply (if employed,; not shown) into a grounded electrical outlet (not shown) of the correct voltage and current rating. Plug the low voltage cable from the power supply into the control unit 40.
2. Turn the control unit 40 on using the power switch on the side. The Power light will come on.

### LOADING THE CARPULE

1. Place dental needle 11 onto the cartridge holder 12.
2. Load an anesthetic cartridge into the cartridge holder 12, pushing it forward so the butt end of the needle punctures the rubber diaphragm of the cartridge, in an otherwise conventional manner.
3. Press the Load button 45 once to advance the plunger and harpoon.
4. Insert the loaded plastic cartridge holder 12 into the handpiece 10, embedding the plunger harpoon 20 into the cartridge plunger or seal 32. Twist the cartridge holder 12 to lock it into the handpiece 10, thereby causing the lugs 82 and lips 83 to removably and physically engage, securing cartridge or carpule holder 12 to handpiece 10 in a bayonet-like manner.

### SELECTING THE INJECTION PROGRAM

The inventive syringe according to the invention divides the delivery of anesthesia into two phases. During the first 10 seconds of the injection, anesthetic is delivered at an extremely slow rate to maximize patient comfort. The injection rate then automatically increases to the preprogrammed rate associated with the injection type you have selected.
1. Select you injection type by depressing the appropriate button. The injection rate will be shown in the Rate (cc/sec) display 42 on the control unit 40.

### USING THE HANDPIECE

1. Once the injection technique is selected, the device 10 is ready to conduct an injection.
2. Aspiration is achieved by pressing the releasing the Aspirate (middle) button 73 on the handpiece 10.
3. After aspiration, press and release the front Start/Stop (forward) button 74 on the handpiece 10 to initiate the injection.
4. At any time during the injection the operator may stop by simply pressing the front Start/Stop button 74 on the handpiece 10 to stop the program.
5. The operator can double the rate of injection during the injection sequenced by pressing the Double (rear) button 75 on the handpiece 10 or the Double button 76 on the control unit 40.
6. When the injection is complete, the operator will press and release the Start/Stop button 74 on the handpiece 10 to stop the program.
7. If another injection is required, steps 2-4 are followed and the program will automatically start over.

### REMOVING THE CARPULE

To remove the cartridge and holder, a user will press the Unload button 45 on the control unit 40 to fully retract the plunger 23. This will pull the harpoon 20 from the cartridge 32. The user will then untwist and remove the loaded cartridge holder 12 (with needle 11 attached) from the handpiece, bu disengaging lugs 82 from lips 83.

### PRE-PROGRAMMED INJECTION RATES

The following table list exemplary pre-programmed injection rates for different dental injection procedures.

| INJECTION TYPE | RATE (CC/SEC) | TYPICAL VOLUME | TYPICAL TIME |
|---|---|---|---|
| Block | .020 | 1.8 cc | 1 min 30 sec |
| Infiltration | .017 | 1.4 cc | 1 min 20 sec |
| PDL | .007 | .2 cc per tooth root | 30 sec |
| AMSA/P-ASA | .008 | .6 cc | 4 min |
| Lingual Infiltration | .010 | 1.4 cc | 2 min 20 sec |

### GENERAL OPERATING TECHNIQUE

The syringe according to the invention allows the delivery of a slow drip of anesthetic that, if not impossible, is very difficult to accomplish with manual, thumb controlled syringes that have been in use for over on hundred years. The syringe according to the invention, with a computer controlled motor, delivers the anesthetic of choice at a programmable constant pressure and volume. Studies show that a slow controlled release of the anesthetic solution keeps the anesthetic in the target zone and does not force the fluid into the surrounding tissue where its effect is drastically reduced. This also minimized the pain associated with injection by minimizing soft tissue trauma that results when a sudden bonus or solution creates undue pressure or tears the tissue. The resulting ease of administration and reduction of pain reduces the patient stress associated with injections for dental anesthesia.

It should therefore be apparent that the dental syringe as described herein carries out the object of the invention and otherwise provides an advance and contribution to the art. The invention has been exemplified with respect to drawings and description, without an attempt to provide a depiction or description of every embodiment. Those skilled in the art will readily understand that various sizes, components and method steps can be employed.

## Claims

1. A dental anesthesic syringe comprising:
(i) a handheld motor driven syringe unit (10) comprising
- a needle (11),
- a cartridge (12) of dental anesthetic and
- means for flowing the dental anesthetic through the needle; and
(ii) a control unit (40) to control operation of the handheld unit, the control unit comprising means for operating the means for flowing the dental anesthetic through the needle to deliver the dental anesthetic through the needle at a first predetermined rate for a first predetermined time and then at a second predetermined rate for a second predetermined time,
**characterised in that** the handheld motor driven syringe unit houses a stepper motor (60) and a lead screw (61) which drives a syringe plunger,

2. The dental anesthesic syringe as in Claim 1, wherein the second predetermined rate is selectable by a user.

3. The dental anesthesic syringe as in claim 1, wherein:
the means for flowing the anesthetic comprises a motor and a plunger, wherein operation of the motor in a first direction advances the plunger against the cartridge of anesthetic to flow anesthetic from the cartridge through the needle and operation of the motor in a second direction retracts the plunger from the cartridge of anesthetic; and
the means for operating the means for flowing further comprises a processing unit to generate control signals to operate the motor to advance and retract the plunger.

4. The dental anesthesic syringe according to any one of the preceding claims further comprising a computer program embodied on a computer readable medium and executable by a microprocessor for controlling the device to deliver a local anesthetic into a patient through a needle, the computer program comprising computer instructions for executing the steps of:
(A) generating a first at least one signal to operate the device to deliver the local anesthetic at a first predetermined rate for a first predetermined time period;
(B) transmitting the first at least one signal to the device to operate the device to deliver the local anesthetic at a first predetermined rate for a first predetermined time period;
(C) generating a second at least one signal to operate the device to deliver the local anesthetic at a second predetermined rate for a second predetermined time period; and
(D) transmitting the second at least one signal to the device for the device to operate at the second predetermined rate for the second predetermined time immediately upon completion of the first predetermined time period.

5. The dental anesthesic syringe according to claim 4 wherein the step of generating a second at least one signal further comprises the steps of:
(C-1) receiving a user signal corresponding to an injection type selected by a user;
(C-2) determining a delivery rate and delivery time based on the injection type received in the user signal; and
(C-3) generating the second at least one signal to operate the device to deliver the local anesthetic at the delivery rate for the delivery time based on the selected injection type.

6. The dental anesthesic syringe according to claim 4 further comprising computer instructions for executing the steps of:
(E-1) calculating a rate of delivery of the local anesthetic through the needle; and
(E-2) displaying the calculated rate of delivery of the local anesthetic.

7. The dental anesthesic syringe according to claim 4 further comprising computer instructions for executing the steps of:
(F-1) calculating an elapsed time of delivery of the local anesthetic through the needle; and
(F-2) displaying the elapsed time of delivery of the local anesthetic.

8. The dental anesthesic syringe according to claim 4 further comprising computer instructions for executing the steps of:
(G-1) calculating a volume of the local anesthetic delivered through the needle; and
(G-2) displaying the volume of delivered local anesthetic.

9. The dental anesthesic syringe according to claim 4 further comprising computer instructions for executing the steps of:
(H-1) receiving a user signal corresponding to a load command selected by a user;
(H-2) generating a third at least one signal to operate the device to position components of the device to receive a cartridge storing the local anesthetic; and
(H-3) transmitting the third at least one signal to the device for loading the local anesthetic cartridge in the device.

10. The dental anesthesic syringe according to claim 9 wherein the step of receiving a user signal corresponding to a load command further comprises the step of receiving the user signal from a control unit.

11. The dental anesthesic syringe according to claim 4 further comprising computer instructions for executing the steps of:
(I-1) receiving a user signal corresponding to an unload command selected by a user,
(I-2) generating a third at least one signal to operate the device to position components of the device to remove a cartridge storing the local anesthetic; and
(I-3) transmitting the third at least one signal to the device for unloading the local anesthetic cartridge from the device.

12. The dental anesthesic syringe according to claim 11 wherein the step of receiving a user signal corresponding to a unload command further comprises the step of receiving the user signal from a control unit.

13. The dental anesthesic syringe according to claim 4 further comprising computer instructions for executing the steps of:
(K-1) receiving a user signal corresponding to a double command selected by a user;
(K-2) generating a third at least one signal to operate the device to increase the rate of delivery of the local anesthetic to twice the rate of delivery being provided by the device; and
(K-3) transmitting the third at least one signal to the device for increasing the rate of delivery of the local anesthetic by the device.

14. The dental anesthesic syringe according to claim 13 wherein the step of receiving a user signal corresponding to a double command further comprises the step of receiving the user signal from the device.

15. The dental anesthesic syringe according to claim 4 wherein the first predetermined time period is 10 seconds.

16. The dental anesthesic syringe according to claim 4 wherein the first predetermined rate is 0.004 cc/sec.

17. The dental anesthesic syringe according to claim 6 wherein the injection type is selected from the group consisting of block, infiltration, PDL, AMSA/P-ASA and lingual infiltration.

18. The dental anesthesic syringe according to claim 4 wherein the second predetermined rate in the range of 0.005 - 0.02 cc/sec.

19. The dental anesthesic syringe according to claim 4 wherein the second predetermined time is in the range of 30 - 240 seconds.

## Patentansprüche

1. Dentalanästhesie-Spritze, die aufweist:
(i) ein motorgetriebenes Spritzen-Handgerät (10), das aufweist:
- eine Nadel (11)
- eine Dentalanästhetikum-Patrone (12) und
- Mittel zum Durchfließen des Dentalanästhetikums durch die Nadel; und
(ii) ein Kontrollgerät (40) zur Kontrolle des Handgerätes, wobei das Kontrollgerät Mittel aufweist zum Betrieb der Mittel zum Durchfließen lassen des Dentalanästhetikums durch die Nadel, um das Dentalanästhetikum durch die Nadel in einer ersten bestimmten Rate während einer ersten bestimmten Zeit abzugeben und dann in einer zweiten bestimmten Rate während einer zweiten bestimmten Zeit;
**dadurch gekennzeichnet, dass** das motorgetriebene Spritzen-Handgerät einen Schrittmotor (60) und eine Verstellschraubenspindel (61) aufweist, die einen Spritzenkolben antreiben.

2. Dentalanästhesie-Spritze nach Anspruch 1, worin die zweite bestimmte Rate durch einen Anwender wählbar ist.

3. Dentalanästhesie-Spritze nach Anspruch 1, worin die Mittel zum Durchfließen lassen des Anästhetikums einen Motor und einen Kolben aufweisen, worin der Betrieb des Motors in einer ersten Richtung den Kolben gegen die Patrone des Anästhetikums vorschiebt, um Anästhetikum aus der Patrone durch die Nadel fließen zu lassen, und der Betrieb des Motors in einer zweiten Richtung den Kolben aus der Patrone des Anästhetikums zurückzieht; und die Einrichtung zum Betrieb der Mittel zum Durchfließen lassen ferner einen Prozessor zur Ausbildung von Kontrollsignalen aufweist, um den Motor zum Vorschieben oder Zurückziehen des Kolbens zu betreiben.

4. Dentalanästhesie-Spritze nach einem der vorhergehenden Ansprüche, die ferner ein Computerprogramm in einem Computer-lesbaren Medium und durch einen Mikroprozessor durchführbar aufweist, zur Steuerung der Vorrichtung, um ein Lokalanästhetikum in einen Patienten durch eine Nadel abzugeben, wobei das Computerprogramm Computerinstruktionen aufweist zur Durchführung der Stufen von:
(A) Ausbilden von mindestens einem ersten Signal, um die Vorrichtung so zu betreiben, dass das Lokaleanästhetikum mit einer ersten bestimmten Rate während einer ersten bestimmten Zeitraums abgegeben wird;
(B) Übertragen des mindestens einen ersten Signals zur Vorrichtung, um die Vorrichtung so zu betreiben, dass sie das Lokalanästhetikum mit einer ersten bestimmten Rate während eines ersten bestimmten Zeitraums abgibt;
(C) Ausbilden von mindestens einem zweiten Signal, um die Vorrichtung so zu betreiben, dass sie das Lokalanästhetikum bei einer zweiten bestimmten Rate während eines zweiten bestimmten Zeitraums abgibt; und
(D) Übertragen des mindestens einen zweiten Signals an die Vorrichtung, um die Vorrichtung bei einer zweiten bestimmten Rate während einer zweiten bestimmten Zeit sofort nach Beendigung des ersten bestimmten Zeitraums zu betreiben.

5. Dentalanästhesie-Spritze nach Anspruch 4, worin die Stufe des Ausbildens von mindestens einem zweiten Signal außerdem die Stufen aufweist:
(C-1) Empfangen eines Anwendersignals, das dem vom Anwender gewählten Injektions-Typ entspricht;
(C-2) Bestimmen der Abgaberate und Abgabezeit auf der Basis des im Anwendersignal empfangenen Injektions-Typs; und
(C-3) Ausbilden des mindestens einen zweiten Signals, um die Vorrichtung so zu betreiben, dass sie das Lokalanästhetikum bei einer Abgaberate während der Abgabezeit auf der Basis des gewählten Injektions-Typs abgibt.

6. Dentalanästhesie-Spritze nach Anspruch 4, die ferner Computerinstruktionen zur Durchführung der Stufen aufweist:
(E-1) Berechnen der Abgaberate des Lokalanästhetikums durch die Nadel und
(E-2) Anzeigen der berechneten Abgaberate des Lokalanästhetikums.

7. Dentalanästhesie-Spritze nach Anspruch 4, die ferner Computerinstruktionen zur Durchführen der Stufen aufweist:
(F-1) Berechnen eines verstrichenen Abgabezeitraums des Lokalanästhetikums durch die Nadel und
(F-2) Anzeigen der verstrichenen Zeit der Abgabe des Lokalanästhetikums.

8. Dentalanästhesie-Spritze nach Anspruch 4, die ferner Computerinstruktionen zur Durchführung der Stufen aufweist:
(G-1) Berechnen des durch die Nadel abgegebenen Volumens des Lokalanästhetikums und
(G-2) Anzeigen des Volumens des abgegebenen Lokalanästhetikums.

9. Dentalanästhesie-Spritze nach Anspruch 4, die ferner Computerinstruktionen zur Durchführung der Stufen aufweist:
(H-1) Empfangen eines Anwendersignals, das einer von einem Anwender ausgewählten Aufladungsanweisung entspricht;
(H-2) Ausbilden von mindestens einem dritten Signal, um die Vorrichtung so zu betreiben, um Komponenten der Vorrichtung so zu positionieren, um eine Patronenspeicherung des Lokalanästhetikums zu erhalten; und
(H-3) Übertragen des mindestens einen dritten Signals an die Vorrichtung zum Aufladen der Patrone für das Lokalanästhetikum in der Vorrichtung.

10. Dentalanästhesie-Spritze nach Anspruch 9, worin die Stufe des Empfangens eines Anwendersignals einer Aufladungsanweisung entspricht, die ferner die Stufe des Empfangens des Anwendersignals von einer Kontrolleinheit umfasst.

11. Dentalanästhesie-Spritze nach Anspruch 4, die ferner Computerinstruktionen zur Durchführung der Stufen umfasst:
(I-1) Empfangen eines Anwendersignals, das einer vom Anwender gewählten Abgabeanweisung entspricht;
(1-2) Ausbilden von mindestens einem dritten Signal, um die Vorrichtung so zu betreiben, dass sie die Komponenten der Vorrichtung so positioniert, dass sie die Patronenspeicherung des Lokalanästhetikums beendet; und
(I-3) Übertragen des mindestens einen dritten Signals zur Vorrichtung, um die Lokalanästhetikumpatrone aus der Vorrichtung abzugeben.

12. Dentalanästhesie-Spritze nach Anspruch 11, worin die Stufe des Empfangens eines Anwendersignals, das der Abgabeanweisung entspricht, außerdem die Stufe des Empfangens des Anwendersignals aus einer Kontrolleinheit umfasst.

13. Dentalanästhesie-Spritze nach Anspruch 4, die ferner Computerinstruktionen zur Durchführung der Stufen aufweist:
(K-1) Empfangen eines Anwendersignals, das einer vom Anwender gewählten doppelten Bedienungsanweisung entspricht;
(K-2) Ausbilden von mindestens einem dritten Signal, um die Vorrichtung so zu betreiben, dass sie die Abgaberate des Lokalanästhetikums auf einen Wert erhöht, der der zweifachen der von der Vorrichtung bereitgestellten Abgaberate entspricht; und
(K-3) Übertragen des mindestens einen dritten Signals zur Vorrichtung, um die Abgaberate des Lokalanästhetikums durch die Vorrichtung zu erhöhen.

14. Dentalanästhesie-Spritze nach Anspruch 13, worin die Stufe des Empfangens eines Anwendersignals, das einer doppelten Bedienungsanweisung entspricht, ferner die Stufe des Empfangens des Anwendersignals von der Vorrichtung umfasst.

15. Dentalanästhesie-Spritze nach Anspruch 4, worin der erste bestimmte Zeitraum 10 Sekunden beträgt.

16. Dentalanästhesie-Spritze nach Anspruch 4, worin die erste bestimmte Rate 0,004 cm³/s beträgt.

17. Dentalanästhesie-Spritze nach Anspruch 6, worin der Injektions-Typ ausgewählt ist aus der Gruppe bestehend aus Blockade, Infiltration, PDL, AMSA/PASA und Lingual-Infiltration.

18. Dentalanästhesie-Spritze nach Anspruch 4, worin die zweite bestimmte Rate im Bereich von 0,005 bis 0,02 cm³/s liegt.

19. Dentalanästhesie-Spritze nach Anspruch 4, worin die zweite bestimmte Zeit im Bereich von 30 bis 240 Sekunden liegt.

## Revendications

1. Seringue d'anesthésie dentaire comprenant :
(i) une unité à main (10) de seringue entraînée par un moteur comportant
- une aiguille (11),
- une cartouche (12) d'anesthésiant dentaire, et
- un moyen d'écoulement de l'anesthésiant dentaire à travers l'aiguille ; et
(ii) une unité de commande (40) destinée à commander le fonctionnement de l'unité à main, l'unité de commande comportant un moyen pour actionner le moyen pour l'écoulement de l'anesthésiant dentaire à travers l'aiguille afin de délivrer l'anesthésiant dentaire à travers l'aiguille à un premier débit prédéterminé pendant un premier temps prédéterminé, puis à un second débit prédéterminé pendant un second temps prédéterminé ;
**caractérisée en ce que** l'unité à seringue à main entraînée par un moteur loge un moteur pas à pas (60) et une vis mère (61) qui entraîne un plongeur de seringue.

2. Seringue d'anesthésie dentaire selon la revendication 1, dans laquelle le second débit prédéterminé peut être sélectionné par un utilisateur.

3. Seringue d'anesthésie dentaire selon la revendication 1, dans laquelle :
le moyen pour l'écoulement de l'anesthésiant comporte un moteur et un plongeur, dans lequel la mise en action du moteur dans un premier sens fait avancer le plongeur contre la cartouche d'anesthésiant pour faire écouler l'anesthésiant depuis la cartouche à travers l'aiguille et l'actionnement du moteur dans un second sens rétracte le plongeur de la cartouche d'anesthésiant ; et le moyen pour actionner le moyen d'écoulement comporte en outre une unité de traitement destinée à générer des signaux de commande pour actionner le moteur afin de faire avancer et de rétracter le plongeur.

4. Seringue d'anesthésie dentaire selon l'une quelconque des revendications précédentes, comportant en outre un programme d'ordinateur matérialisé dans un support lisible par ordinateur et pouvant être exécuté par un microprocesseur pour commander le dispositif afin de délivrer un anesthésiant local à un patient par l'intermédiaire d'une aiguille, le programme d'ordinateur comprenant des instructions d'ordinateur pour l'exécution des étapes qui consistent :
(A) à générer au moins un premier signal pour actionner le dispositif afin de délivrer l'anesthésiant local à un premier débit prédéterminé pendant une première période de temps prédéterminée ;
(B) à transmettre le, au moins un, premier signal au dispositif pour actionner le dispositif afin de délivrer l'anesthésiant local à un premier débit prédéterminé pendant une première période de temps prédéterminée ;
(C) à générer au moins un second signal pour actionner le dispositif afin de délivrer l'anesthésiant local à un second débit prédéterminé pendant une seconde période de temps prédéterminée ; et
(D) à transmettre le, au moins un, second signal au dispositif pour faire fonctionner le dispositif au second débit prédéterminé pendant la seconde période de temps prédéterminée dès la fin de la première période de temps prédéterminée.

5. Seringue d'anesthésie dentaire selon la revendication 4, dans laquelle l'étape de génération d'au moins un second signal comprend en outre les étapes qui consistent :
(C-1) à recevoir un signal d'utilisateur correspondant à un type d'injection sélectionné par un utilisateur ;
(C-2) à déterminer un débit de délivrance et un temps de délivrance sur la base du type d'injection reçu dans le signal d'utilisateur ; et
(C-3) à générer le, au moins un, second signal pour actionner le dispositif afin de délivrer l'anesthésiant local au débit de délivrance pendant le temps de délivrance basé sur le type d'injection sélectionnée.

6. Seringue d'anesthésie dentaire selon la revendication 4, comportant en outre des instructions d'ordinateur pour l'exécution des étapes qui consistent :
(E-1) à calculer un débit de délivrance de l'anesthésiant local à travers l'aiguille ; et
(E-2) à afficher le débit de délivrance calculée de l'anesthésiant local.

7. Seringue d'anesthésie dentaire selon la revendication 4, comportant en outre des instructions d'ordinateur pour l'exécution des étapes qui consistent :
(F-1) à calculer un temps écoulé de délivrance de l'anesthésiant local à travers l'aiguille ; et
(F-2) à afficher le temps de délivrance écoulé de l'anesthésiant local.

8. Seringue d'anesthésie dentaire selon la revendication 4, comportant en outre des instructions d'ordinateur pour l'exécution des étapes qui consistent :
(G-1) à calculer un volume de l'anesthésiant local délivré à travers l'aiguille ; et
(G-2) à afficher le volume de l'anesthésiant local délivré.

9. Seringue d'anesthésie dentaire selon la revendication 4, comportant en outre des instructions d'ordinateur pour l'exécution des étapes qui consistent :
(H-1) à recevoir un signal d'utilisateur correspondant à un ordre de chargement sélectionné par un utilisateur ;
(H-2) à générer au moins un troisième signal pour actionner le dispositif afin de positionner des constituants du dispositif pour recevoir une cartouche emmagasinant l'anesthésiant local ; et
(H-3) à transmettre le, au moins un, troisième signal au dispositif pour le chargement de la cartouche d'anesthésiant local dans le dispositif.

10. Seringue d'anesthésie dentaire selon la revendication 9, dans laquelle l'étape de réception d'un signal d'utilisateur correspondant à un ordre de chargement comprend en outre l'étape de réception du signal d'utilisateur depuis une unité de commande.

11. Seringue d'anesthésie dentaire selon la revendication 4, comprenant en outre des instructions d'ordinateur pour l'exécution des étapes qui consistent :
(I-1) à recevoir un signal d'utilisateur correspondant à un ordre de déchargement sélectionné par un utilisateur ;
(I-2) à générer au moins un troisième signal pour actionner le dispositif afin de positionner des constituants du dispositif pour enlever une cartouche emmagasinant l'anesthésiant local ; et
(1-3) à transmettre le, au moins un, troisième signal au dispositif pour décharger la cartouche d'anesthésiant local du dispositif.

12. Seringue d'anesthésie dentaire selon la revendication 11, dans laquelle l'étape de réception d'un signal d'utilisateur correspondant à un ordre de déchargement comprend en outre l'étape de réception du signal d'utilisateur depuis une unité de commande.

13. Seringue d'anesthésie dentaire selon la revendication 4, comprenant en outre des instructions d'ordinateur pour l'exécution des étapes qui consistent :
(K-1) à recevoir un signal d'utilisateur correspondant à un ordre double sélectionné par un utilisateur ;
(K-2) à générer au moins un troisième signal pour actionner le dispositif afin d'augmenter le débit de délivrance de l'anesthésiant local, du double du débit de délivrance fourni par le dispositif ; et
(K-3) à transmettre le, au moins un, troisième signal au dispositif pour augmenter le débit de délivrance de l'anesthésiant local par le dispositif.

14. Seringue d'anesthésie dentaire selon la revendication 13, dans laquelle l'étape de réception d'un signal d'utilisateur correspondant à un ordre double comprend en outre l'étape de réception du signal d'utilisateur depuis le dispositif.

15. Seringue d'anesthésie dentaire selon la revendication 4, dans laquelle la première période de temps prédéterminée est de 10 secondes.

16. Seringue d'anesthésie dentaire selon la revendication 4, dans laquelle le premier débit prédéterminé est de 0,004 cm³/s.

17. Seringue d'anesthésie dentaire selon la revendication 6, dans laquelle le type d'injection est sélectionné dans le groupe constitué d'un bloc, d'une infiltration, de PDL, de AMSA/P-ASA et d'une infiltration linguale.

18. Seringue d'anesthésie dentaire selon la revendication 4, dans laquelle le second débit prédéterminé est compris dans la plage de 0,005 à 0,02 cm³/s.

19. Seringue d'anesthésie dentaire selon la revendication 4, dans laquelle le second temps prédéterminé est compris dans la plage de 30 à 240 secondes.
